# EUROPEAN PATENT SPECIFICATION

(11) **EP 3 842 098 B1**
(45) Date of publication and mention of the grant of the patent: **09.12.2026**
(21) Application number: 19852898.6
(22) Date of filing: 06.08.2019
(51) Int. Cl.: A61N 5/10, A61B 6/00

(54) **RADIOTHERAPY APPARATUS**
STRAHLENTHERAPIEGERÄT
APPAREIL DE RADIOTHÉRAPIE

(30) Priority: 24.08.2018 CN 201810971120
(43) Date of publication of application: 30.06.2021
(73) Proprietor: Our United Corporation, Xi'an, Shaanxi 710018 (CN)
(72) Inventor: LIU, Haifeng, Xi'an City, Shaanxi 710018 (CN); LI, Daliang, Xi'an City, Shaanxi 710018 (CN)
(74) Representative: Kraus & Lederer PartGmbB
(86) International application number: PCT/CN2019/099397
(87) International publication number: WO 2020/038225

(56) References cited:
- WO-A1-2012/011083
- WO-A1-2017/020244
- CN-A- 101 195 058
- CN-A- 101 195 058
- CN-A- 101 195 058
- CN-A- 109 011 217
- CN-A- 109 011 219
- CN-A- 109 157 762
- CN-Y- 2 258 407
- CN-Y- 2 258 407
- CN-Y- 2 590 600
- CN-Y- 2 590 600
- US-A1- 2015 202 465

## Description

### TECHNICAL FIELD

The present disclosure relates to the field of medical technologies, and more particularly, to a radiotherapy device.

### BACKGROUND

With the development of medical technologies, radiation treatment is more and more widely used in the treatment of tumors.

The existing radiation treatment system for treating heads mainly includes a head gamma knife, which uses a natural isotopic radioactive source, cobalt-60, to emit gamma rays, and uses the radioactivity of the rays to kill tumor cells. However, the rays may also damage normal tissues or cells. In the related technologies, the existing head gamma knife includes 30 or 180 radioactive sources, beams are emitted by a plurality of radioactive sources from different directions and focus on a common focus, such that the common focus has the largest gamma-ray dose rate, whereas the beams emitted from each radioactive source cause less damage to the normal tissues or cells. In this way, an objective of killing the tumor cells while protecting the normal tissues or cells is achieved, thereby achieving tumor treatment effects.

However, in the treatment of head tumors, sensitive tissues and organs (such as the eyes and other important nerves) need to be avoided. In existing radiotherapy devices, radiation of the sensitive tissues and organs is avoided by adjusting a gamma angle for a patient, i.e., by adjusting an elevation angle of head.

The present disclosure provides a new implementation mode for protection of sensitive tissues and organs.

The present disclosure provides a new implementation mode for protection of sensitive tissues and organs.

CN101195058A is directed to a radiotherapy apparatus. The radiotherapy apparatus includes a shielding body, a source body provided on the shielding body, an end collimator provided on the shielding body, a shielding body, an intermediate collimator between the end collimator and the source body. The source body, the end collimator and the intermediate collimator can independently rotate relative to the same rotary center line. The end collimator includes multiple groups of through holes with different apertures, and the intermediate collimator includes: at least one group of through holes, at least one group of through holes in high latitude regions, or at least one group of through holes in low latitude regions.

CN2258407Y is directed to a collimation focusing and shielding device for a cobalt-60 rotary stereo-tactic radiotherapy apparatus for heads. A hollow hemispherical radioactive source body, an intermediate collimation or shielding switch, a rotary helmet, a shielding cover and a magnetic moving shielding block are orderly arranged in the hollow hemisphere of the external shielding body. the screening cover and the magnetic moving screening block are arranged in the helmet. A plurality of radial direction holes pointing to spherical center are distributed in a certain regulation on the radioactive source body, the intermediate collimation or shielding r switch and the spherical surface of the helmet.

CN2590600Y is directed to a medical rotary gamma-ray radiation unit, which comprises a ray shielding body, a source body, and a collimation body. The source body is provided with radiation sources and ray paths, and rays emitted from the radiation sources pass through the ray paths and are aligned to a common focus in the radical direction; the axle wire of a radiation unit is also provided with a rotatable central shaft, the source body is provided with a plurality of groups of radiation sources, each group comprises a plurality of the radiation sources which are distributed in the latitudinal direction between 10° to 50°, the latitude of corresponding points of each adjacent two groups is distributed in the arithmetic progression, and the latitude of each adjacent two points in each group is also distributed in the arithmetic progression; the radiation sources are distributed between 0° and 360° in the longitudinal direction.

WO2017020244A1 is directed to a focussed radiotherapy apparatus and a radiation therapy device. The radiotherapy apparatus at least comprises: a source body, provided with a plurality of radiation sources; a pre-collimation body, fixedly connected to the source body, the pre-collimation body comprising a plurality of pre-collimation holes, the pre-collimation holes corresponding to the radiation sources in a one-to-one manner; a collimation body, covering the source body and the pre-collimation body and rotating with respect to the source body and the pre-collimation body, the collimation body comprising at least one curved surface, a plurality of groups of collimators, having different specifications, and shielding positions being arranged on the curved surface, each group of collimators and/or shielding positions being arranged in correspondence with the radiation sources, the collimation body rotating to switch different groups of collimators and/or shielding positions to be aligned with the radiation sources so as to obtain different field sizes and/or implement at least partial shielding of the source body; a switch body, arranged on an outer side of the collimation body and moving with respect to the source body, the pre-collimation body and the collimation body so as to enable rays of the radiation sources to be focussed or shielded.

US2015202465A1 is directed to a source switching apparatus comprising a plurality of collimators, a switch carrier, a source carrier. The switch carrier is assembled with a plurality of shielding elements regularly distributed thereon. The source carrier is assembled with a plurality of radiation sources regularly distributed thereon. The collimator, the switch carrier, and the source carrier are connected to one another via respective driver, to rotate with common angular velocity or in a differential movement, and wherein in the case when the differential movement is holding, an angle difference is formed between the switch carrier and the source carrier.

### SUMMARY

The invention relates to a system as defined in the appended claims. Embodiments, examples or aspects in the following disclosure, in particular methods, which do not fall under the scope of the claims are presented for illustration purposes only and do not form part of the invention.

The present disclosure provides a radiotherapy device, which can protect sensitive tissues and organs during treatment. The technical solutions are described as follows.

In one aspect, there is provided a collimator, which is applied to a radiotherapy device. The radiotherapy device includes a plurality of radioactive sources, a plurality of collimating hole groups are arranged on the collimator, and an included angle of each of the collimating hole groups in a longitudinal direction is within a preset included angle range.

Each of the collimating hole groups includes a plurality of collimating holes, and beams emitted from the plurality of radioactive sources intersect at a common focus after passing through each collimating hole of the collimating hole group.

In another aspect, there is provided a radiotherapy device, which includes a radioactive source apparatus. The radioactive source apparatus includes a source body and the collimator provided by the present disclosure, and the source body is provided with a plurality of radioactive sources.

A radiotherapy device for treating head tumor as recited in claims 1-10 is provided.

The technical solutions provided by the present disclosure have the following beneficial effects.

The present disclosure provides a radiotherapy device. A plurality of radioactive sources are arranged on the radiotherapy device, a plurality of collimating hole groups are arranged on the collimator, and an included angle of each of the collimating hole groups in a longitudinal direction is within a preset included angle range. Each of the collimating hole groups includes a plurality of collimating holes, and beams emitted from the plurality of radioactive sources intersect at a common focus after passing through each collimating hole of the collimating hole group. The plurality of radioactive sources may be driven by the radiotherapy device to rotate along a central axis of the radiotherapy device, such that the radioactive sources can be turned off when passing through sensitive tissues or organs, and that the radioactive sources can be turned on by emitting beams from the collimating holes on the collimator when passing through normal tissues and organs. In this way, during the treatment of head tumors, the sensitive tissues and organs such as eyes can be protected from extra damage.

### BRIEF DESCRIPTION OF THE DRAWINGS

To describe the technical solutions of the embodiments of the present disclosure more clearly, the accompanying drawings required for describing the embodiments will be briefly introduced below. Apparently, the accompanying drawings in the following description are merely some embodiments of the present disclosure. To those of ordinary skills in the art, other accompanying drawings may also be derived from these accompanying drawings without creative efforts.
FIG. 1 is a schematic structural diagram of an existing radiotherapy device;
FIG. 2 is a schematic structural diagram of an existing radioactive source apparatus;
FIG. 3 is a schematic structural top view of an existing source body;
FIG. 4 is a schematic structural top view of an existing collimator according to an embodiment not covered by the claimed invention;
FIG. 5 is a schematic diagram of another collimator according to an embodiment not covered by the claimed invention;
FIG. 6 is a schematic diagram of still another collimator according to an embodiment not covered by the claimed invention;
FIG. 7 is a schematic diagram of a radiotherapy device according to an embodiment of the present disclosure;
FIG. 8 is a schematic diagram of another radiotherapy device according to an embodiment of the present disclosure;
FIG. 9 is a schematic diagram of still another radiotherapy device according to an embodiment of the present disclosure;
FIG. 10 is a schematic diagram of still another radiotherapy device according to an embodiment of the present disclosure;
FIG. 11 is a schematic diagram of still another collimator according to an embodiment of the present disclosure;
FIG. 12 is a schematic diagram of a control driving method according to an embodiment not covered by the claimed invention;
FIG. 13 is a schematic diagram of a treatment irradiation according to an embodiment of the present disclosure;
FIG. 14 is a schematic diagram of another control driving method according to an embodiment not covered by the claimed invention;
FIG. 15 is a schematic diagram of another treatment irradiation according to an embodiment not covered by the claimed invention;
FIG. 16 is a schematic diagram of still another control driving method according to an embodiment not covered by the claimed invention;
FIG. 17 is a schematic diagram of still another control driving method according to an embodiment not covered by the claimed invention; and
FIG. 18 is a schematic diagram of still another control driving method according to an embodiment not covered by the claimed invention.

### DETAILED DESCRIPTION OF THE EMBODIMENTS

Detailed description of implementations of the present disclosure will further be made below with reference to drawings in order to make the above objectives, technical solutions and advantages of the present disclosure more apparent.

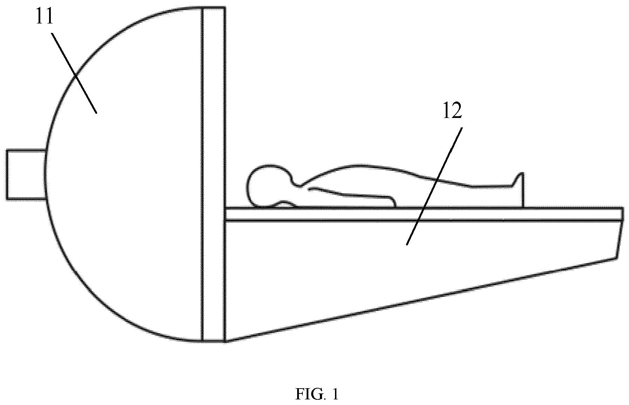

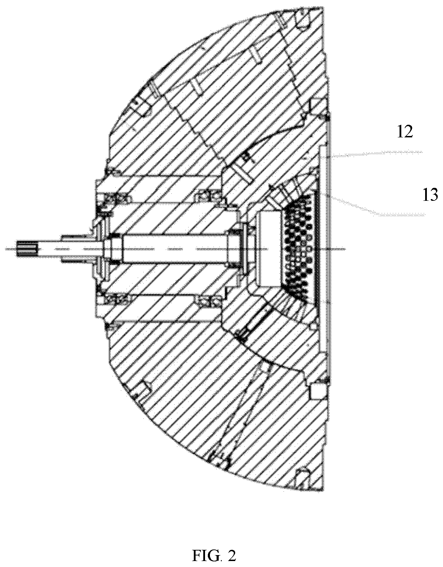

An existing radiotherapy device for treatment of head tumors is provided. Principles for radiation treatment of head tumors are as shown in FIG. 1 and FIG. 2. A plurality of radioactive sources may be mounted in a source body 12. Beams emitted from the plurality of radioactive sources intersect at a common focus after passing through a collimating hole on a collimator 13, and the common focus is located in a cavity of a radioactive source apparatus 11 of the radiotherapy device. A treatment couch 12 is used to carry a patient and to move the patient into a treatment cabin of the radioactive source apparatus 10, such that a nidus in the patient is located at the common focus for radiation treatment.

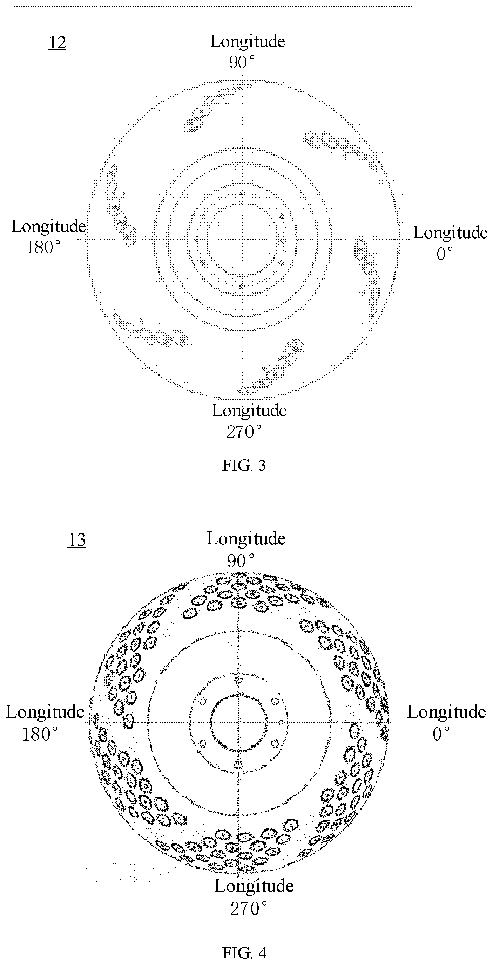

The source body 12 of the existing radiotherapy device is bowl-shaped as shown in FIG. 3. The radioactive sources may be divided into six groups, wherein each group includes five radioactive sources, i.e., a total of 30 radioactive sources. The 30 radioactive sources are distributed on the source body. As shown in FIG. 4, the collimator 13 includes six collimation channel groups. The six collimation channel groups correspond to the positions of the six groups of radioactive sources. Each collimation channel group includes four subgroups. Collimating holes of one subgroup are filled with solid tungsten rods to implement off source shielding. The other subgroups each include five collimating holes, and the collimating holes in different subgroups have different sizes.

During treatment, the source body and the collimator may be driven to rotate with respect to each other to switch the collimating holes of different sizes and to implement on/off source by shielding the radioactive sources by the collimator. However, the six groups of collimating holes having different sizes and the on/off source are switched simultaneously, and one of the groups cannot be controlled individually. Therefore, during the treatment, a gamma angle needs to be adjusted, such that rays are kept away from sensitive tissues and organs such as eyes.

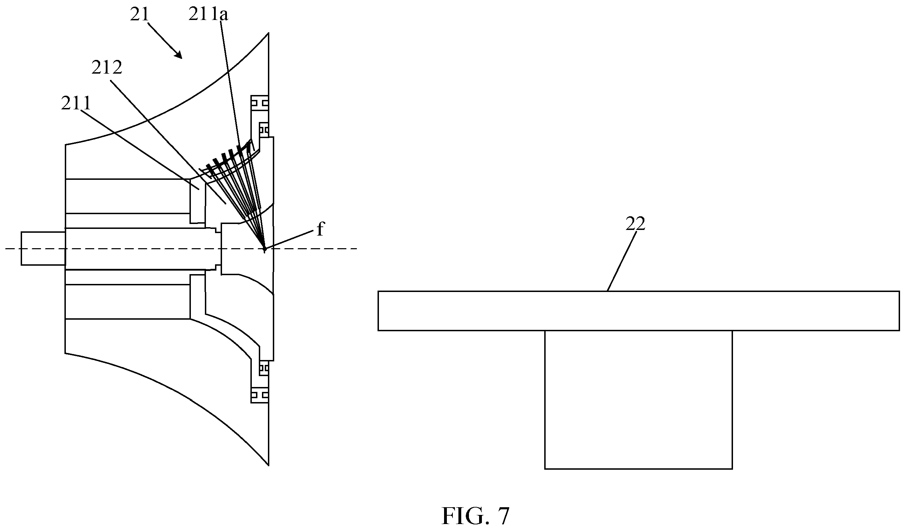

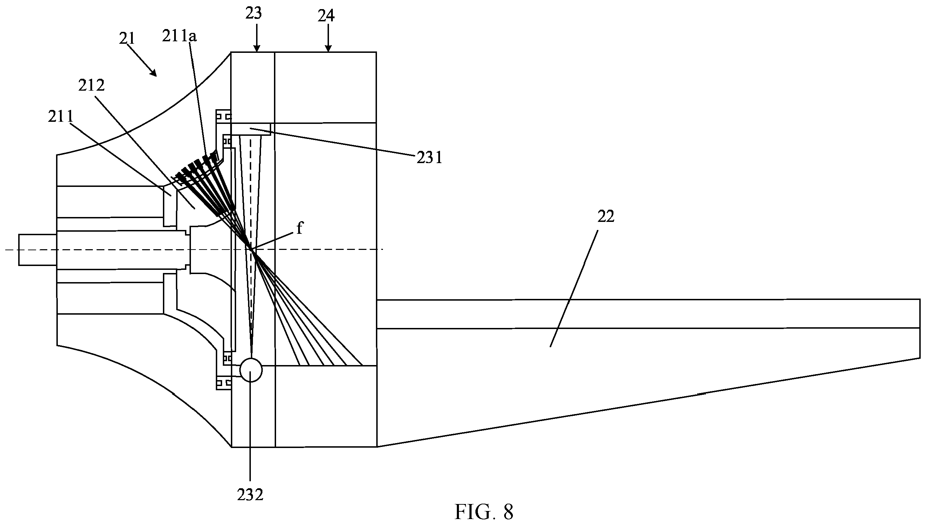

As shown in FIG. 7 and FIG. 8, the present disclosure provides a radiotherapy device, which includes a radioactive source apparatus 21. The radioactive source apparatus 21 includes a source body 211 and a collimator 212, the source body 211 is provided with a plurality of radioactive sources 211a, the collimator 212 is provided with a plurality of collimating hole groups, and an included angle of each of the collimating hole groups in a longitudinal direction is within a preset included angle range. Each of the collimating hole groups includes a plurality of collimating holes, and beams emitted from the plurality of radioactive sources intersect at a common focus f after passing through each collimating hole of the collimating hole group.

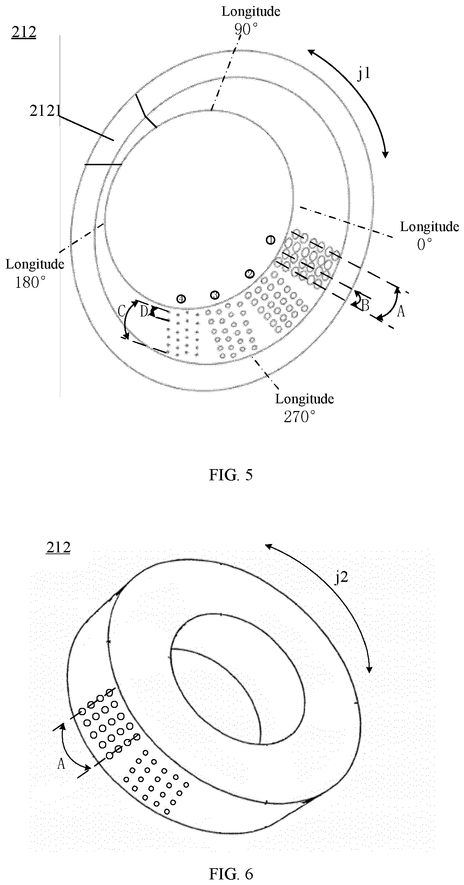

For example, as shown in FIG. 5, the collimator 212 may be bowl-shaped, and the longitudinal direction of the collimator 212 is as shown by Arrow j1 in FIG. 5, which is 0°-360° in the longitudinal direction. The collimator 212 may also be tube-shaped as shown in FIG. 6, and the longitudinal direction of the collimator 212 is the direction as shown by Arrow j2 in FIG. 6. Two ends of a round table in FIG. 6 have equal size, or of course, may have different sizes. The present disclosure does not limit the specific shape of the collimator, and the longitudinal direction in the present disclosure is described only by taking FIG. 5 and FIG. 6 as examples.

In the present disclosure, the included angle between the collimating hole groups in the longitudinal direction is within a preset included angle range, and the maximum included angle between the plurality of collimating hole groups in the longitudinal direction is within the preset included angle range. Moreover, in the present disclosure, as shown in FIG. 5, the included angle between the collimating bodies 212 in the longitudinal direction is an included angle formed by using centers of the collimating bodies 212 as a reference. It is to be noted here that if the collimating bodies include one row and the centers of the plurality of collimating bodies in the same row are located on the same longitudinal line, it is believed that the included angle between the plurality of collimating bodies in the longitudinal direction is zero degree. In the present disclosure, the preset included angle range is greater than or equal to zero degree.

For example, as shown in FIG. 5, the present disclosure provides a collimator 212. The collimator 212 in FIG. 5 is provided with four collimating hole groups as an example, i.e., No. 1collimating hole group, No. 2collimating hole group, No. 3collimating hole group, and No. 4collimating hole group. Each collimating hole group includes 24 collimating holes respectively as an example. Taking the No. 1collimating hole group as an example, beams emitted from the plurality of radioactive sources intersect at a common focus after passing through each collimating hole of the No. 1collimating hole group. The included angle of the No. 1collimating hole group, the included angle of the No. 2collimating hole group, the included angle of the No. 3collimating hole group, and the included angle of the No. 4collimating hole group in the longitudinal direction are within the preset included angle range. In FIG. 5, the No. 1collimating hole group is taken as an example, and the included angle of the No. 1 collimating hole group in the longitudinal direction (the direction as shown by Arrow j1 in FIG. 5) is denoted as A. As an example, the preset included angle range A may be between 5° and 60°, i.e., 5°≤A≤60°. The preset included angle range A may be any included angle in the range of from 5° to 60°. As an example, the preset included angle range A may be 5°, 8°, 10°, 12°, 18°, 20°, 25°, 30°, 40°, 45°, 50°, or 60°. Reference may be made to other descriptions of the present disclosure for specific description of the collimator.

The collimator is provided with a plurality of collimating hole groups, which may mean that the collimator is provided with two or more collimating hole groups. In FIG. 5, an exemplary description is made by taking an example where the collimator 212 is provided with four collimating hole groups. Each collimating hole group includes a plurality of collimating holes, and the number of the plurality of collimating holes corresponding to the radioactive sources may range from 20 to 180, for example, 30 or 180. The present disclosure does not limit the number or arrangement of the collimating holes, and the example as shown in FIG. 5 merely serve as an exemplary description. The radiotherapy device also includes a plurality of radioactive sources. The collimating holes on the collimator correspond to the radioactive sources in number and arrangement, such that the beams emitted from the radioactive sources intersect at a common focus after passing through the collimating holes.

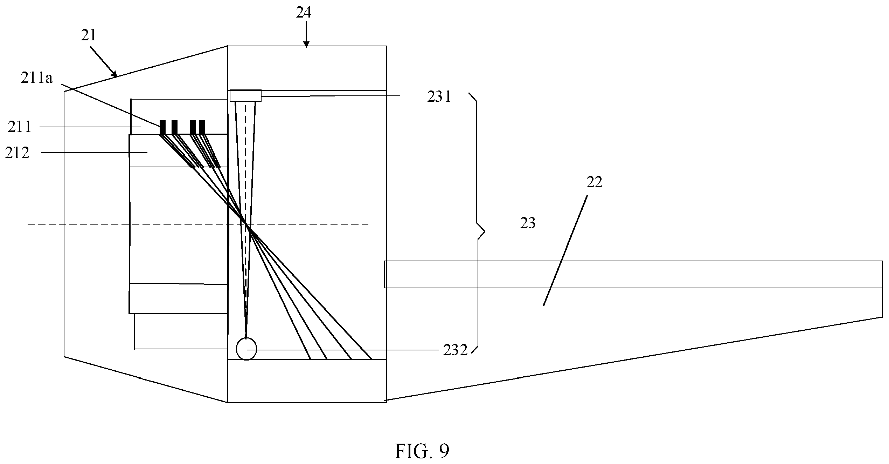

For example, as shown in FIG. 6, the collimator 212 may also be tube-shaped as shown in FIG. 6, and the longitudinal direction of the collimator 212 is the direction as shown by Arrow j2 in FIG. 6. Two ends of a round table in FIG. 6 have equal size, or of course, may have different sizes (as shown in FIG. 9). The present disclosure does not limit the specific shape of the source body, and the shapes as shown in FIG. 5 and FIG. 6 are taken as examples. An exemplary description is made in FIG. 6 by taking an example where the collimator is provided with two collimating hole groups and each collimating hole group includes 20 collimating holes.

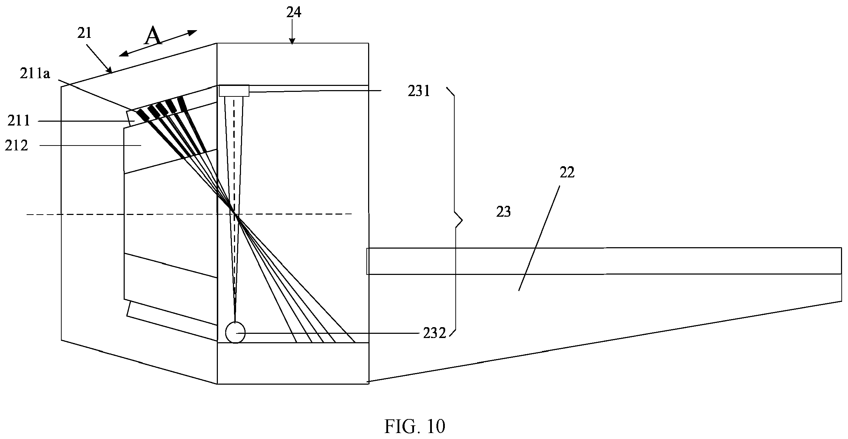

As an example, in the radiotherapy device as shown in FIG. 9, the source body 211 is tube-shaped, and two ends of the tube-shaped source body 211 have equal diameter. In the radiotherapy device as shown in FIG. 9, the source body 211 and/or the collimator 212 may also move in a direction as shown by the central axis of the radioactive source apparatus 21 to implement on/off source. For example, in the radiotherapy device as shown in FIG. 10, the source body 211 is tube-shaped, and two ends of the tube-shaped source body 211 have unequal diameters. In the radiotherapy device as shown in FIG. 10, the source body 211 and/or the collimator 212 may also move in Direction A to implement on/off source. Of course, on/off source may be implemented through rotation, which is not limited in the present disclosure.

The present disclosure provides a radiotherapy device. A plurality of radioactive sources 211a are arranged on the radiotherapy device, a plurality of collimating hole groups are arranged on the collimator, and an included angle of each of the collimating hole groups in a longitudinal direction is within a preset included angle range. Each of the collimating hole groups includes a plurality of collimating holes, and beams emitted from the plurality of radioactive sources 211a intersect at a common focus after passing through each collimating hole of the collimating hole group. The plurality of radioactive sources 211a may be driven by the radiotherapy device to rotate along a central axis of the radiotherapy device, such that the radioactive sources 211a can be turned off when passing through sensitive tissues or organs, and that the radioactive sources 211a can be turned on by emitting beams from the collimating holes on the collimator when passing through normal tissues and organs. In this way, during the treatment of head tumors, the sensitive tissues and organs such as eyes can be protected from extra damage.

In the radiotherapy device provided by the present disclosure, the radioactive source apparatus further includes a source body driving apparatus configured to drive the radioactive source to rotate about a central axis thereof. The driving apparatus may be a motor, and the radioactive source apparatus may also monitor the driving of the motor to obtain the relative position of the radioactive source in real time so as to determine whether to turn on or off the radioactive source.

During the treatment, a tumor of a patient may be accurately located at a common focus, such that tumor cells may be killed by radioactive rays. However, if the patient is moved during the treatment, the radioactive rays may deflect, which not only is disadvantageous to the treatment but also is harmful to health of the patient. The common focus of the existing radiotherapy device is located in a cavity of the radioactive source apparatus, and thus it is impossible to monitor whether the patient's head moves during the treatment. In the radiotherapy device provided by the present disclosure, the common focus is located outside an end surface of the radioactive source apparatus. For example, as shown in FIG. 8-FIG. 10, the common focus is located outside the end surface of the radioactive source apparatus, which is advantageous to observe and monitor whether the patient is moved during the treatment.

In the radiotherapy device provided by the present disclosure, the radiotherapy device also includes an imaging apparatus 23. The imaging apparatus 23 is arranged on a side of the radioactive source apparatus 21, and the common focus f is located within an imaging region of the imaging apparatus 23. That is, a tumor of a patient within the imaging region is imaged by the imaging apparatus 23, so to determine whether the patient is moved on the basis of the image. Displacement monitoring based on images has higher accuracy.

For example, the imaging apparatus 23 in the present disclosure may be any combination of one or more of an X-ray imaging apparatus, a CT (Computed Tomography) imaging apparatus, an MR (Magnetic Resonance) imaging apparatus, a DSA (Digital Subtraction Angiography) imaging apparatus, an ultrasound imaging apparatus, or a PET (Positron Emission Computed Tomography) imaging apparatus. For example, the imaging apparatus is the X-ray imaging apparatus. As an example, as shown in FIG. 8, the imaging apparatus may include an X-ray tube and a flat panel detector. Alternatively, the imaging apparatus may include two X-ray tubes and two flat panel detectors, and beams emitted from the two X-ray tubes intersect. Of course, the imaging apparatus may also be a combination of any two or more different imaging apparatuses. For example, the imaging apparatus may be a combination of the X-ray imaging apparatus and the DSA imaging apparatus. The present disclosure does not limit a specific setup mode of the imaging apparatus, and only uses the above imaging apparatus as an example to make an exemplary description. Specifically, when the imaging apparatus includes an imaging center point, the common focus coincides with the imaging center point.

As shown in FIG. 8, the imaging apparatus 23 may be a bulb tube 232 and a flat panel detector 231 separately provided with a fixing device to fix the imaging apparatus 23, or the imaging apparatus 23 may also be separately provided with a driving apparatus to drive the bulb tube 232 and the flat panel detector 32 to rotate. Alternatively, as shown in FIG. 9, the imaging apparatus 23 may be fixedly arranged in a shielding apparatus 24, the specific position and structure of the shielding apparatus 24 are not limited in the present disclosure, and the above shielding apparatus is used as an example to make an exemplary description.

In addition, the radiotherapy device further includes a treatment couch 22 for carrying a patient. As shown in FIG. 7-FIG. 10, the present disclosure does not limit the specific structure or movement form of the treatment couch 22, which may be a three-dimensional couch as shown in FIG. 8-FIG. 10, or may be a six-dimensional couch as shown in FIG. 7. The treatment couch 22 may be selectively configured according to treatment needs, and related details are omitted herein.

In the radiotherapy device provided by the present disclosure, an anti-sinking component is also arranged between the collimator and the source body. Referring to FIG. 7 to FIG. 8, the anti-sinking component is a bearing.

In the radiotherapy device provided by the present disclosure, the radioactive source apparatus further includes a shielding apparatus 24. The shielding apparatus 24 is located on a side of the radioactive source apparatus 21. Beams emitted from the radioactive source 211a are shielded by the shielding apparatus 24 after passing through the common focus f. For example, as shown in FIG. 8-FIG. 10, the shielding apparatus 24 is located at a side of the common focus f of the radioactive source apparatus 21, and the beams emitted from the radioactive source 211a are shielded by the shielding apparatus 24 after passing through the common focus f, so as to avoid excessive radiation in a treatment room. For example, if the shielding apparatus 24 is shaped like an annular body, all the rays from the radioactive source 211a rotating about the central axis are received by the shielding apparatus. Alternatively, the shielding apparatus 24 is a shielding block that can rotate along the central axis of the radioactive source apparatus 21 to follow the radioactive source 211a to rotate so as to receive the rays after passing through the common focus f. It is to be noted that when the treatment couch 22 carries the patient to move, the shielding apparatus 24 is provided with a channel to facilitate the movement of the treatment couch.

The collimator in the present disclosure is specifically introduced and described below.

The present disclosure provides a collimator applied to a radiotherapy device. The radiotherapy device includes a plurality of radioactive sources, a plurality of collimating hole groups are arranged on the collimator, and an included angle of each collimating hole group in the longitudinal direction is within a preset included angle range. Each of the collimating hole groups includes a plurality of collimating holes, and beams emitted from the plurality of radioactive sources intersect at a common focus after passing through each collimating hole of the collimating hole group.

As shown in FIG. 5, the collimator 212 may be bowl-shaped, and the longitudinal direction of the collimator 212 is as shown by Arrow j1 in FIG. 5, which is 0°-360° in the longitudinal direction. The collimator may also be tube-shaped as shown in FIG. 6, and the longitudinal direction of the collimator is the direction as shown by Arrow j2 in FIG. 6. Two ends of a round table in FIG. 6 have equal size, or of course, may have different sizes (as shown in FIG. 10). The present disclosure does not limit the specific shape of the collimator, and the longitudinal direction in the present disclosure is described only taking the above shapes as examples.

For example, as shown in FIG. 5, the present disclosure provides a collimator 212. The collimator in FIG. 5 is provided with four collimating hole groups as an example, i.e., No. 1collimating hole group, No. 2collimating hole group, No. 3collimating hole group, and No. 4collimating hole group. Each collimating hole group includes 24 collimating holes respectively as an example. Taking the No. 1 collimating hole group as an example, beams emitted from the plurality of radioactive sources intersect at a common focus after passing through each collimating hole of the No. 1collimating hole group. The included angle of the No. 1 collimating hole group, the included angle of the No. 2collimating hole group, the included angle of the No. 3collimating hole group, and the included angle of the No. 4collimating hole group in the longitudinal direction are within the preset included angle range. In FIG. 5, the No. 1collimating hole group is taken as an example, and the included angle of the No. 1collimating hole group in the longitudinal direction (the direction as shown by Arrow j1 in FIG. 5) is denoted as A. As an example, the preset included angle range A may be between 5° and 60°, i.e., 5°≤A≤60°. The preset included angle range A may be any included angle in the range of from 5° to 60°. As an example, the preset included angle range A may be 5°, 8°, 10°, 12°, 18°, 20°, 25°, 30°, 40°, 45°, 50°, or 60°.

For the collimator provided by the present disclosure, in the longitudinal direction, a pitch between two adjacent collimating holes is larger than a size of the radioactive source. Furthermore, the collimator may misalign the radioactive source by a smaller angle, such that the radioactive source can be shielded by a pitch between the collimating holes instead of by a shield. Therefore, the radioactive source can be quickly turned on or off.

For example, for the collimator provided by the present disclosure, in the longitudinal direction, each collimating hole group includes a plurality of rows of radioactive sources, and the included angle between two adjacent rows of radioactive sources ranges from 2° to 15°. For example, among the plurality of collimating hole groups, the included angle between any two adjacent rows of collimating hole groups is the same, or the included angles between two adjacent rows of different collimating hole groups are different, which is not limited in the present disclosure, and what is shown in FIG. 5 is just an exemplary description. As shown in FIG. 5, the plurality of radioactive sources are divided into four rows, taking an example where the included angle between adjacent rows of collimating holes is B (in FIG. 4, two rows are taken as an example), the included angle B may range from 2° to 15°, i.e., 2° ≤B≤15°, and the preset included angle B may be any included angle in the range of from 2° to 15°. For example, the preset included angle range B may be 2°, 2.5°, 3°, 5°, 6°, 8°, 10°, 12°, or 15°.

For the collimator provided by the present disclosure, the included angle between the collimating hole groups in the latitudinal direction ranges from 20° to 60°. For example, as shown in FIG. 5, the preset included angle range C may be between 20° and 60°, i.e., 20° ≤C≤60°, and the preset included angle range C may be any included angle in the range of from 20° to 60°. For example, the preset included angle range C may be 20°, 25°, 30°, 38°, 40°, 45°, 50°, 53°, or 60°.

For example, in the collimator provided by the present disclosure, in the latitudinal direction, the included angle between any two adjacent collimating holes ranges from 1° to 10°. For example, the included angle between any two adjacent collimating holes in the latitudinal direction is the same, or the included angle between any two adjacent collimating holes in the latitudinal direction is different, which is not limited in the present disclosure, and the above example as shown in FIG. 5 merely serves as an exemplary description. For example, as shown in FIG. 5, taking two collimating holes as an example, an included angle between the two collimating holes in the latitudinal direction is denoted as D, the included angle D may range from 1° to 10°, i.e., 1° ≤D≤10°, and the preset included angle D may be any included angle in the range of from 1° to 10°. For example, the preset included angle range D may be 1°, 2°, 3°, 5°, 6°, 8°, 9°, or 10°.

For the collimator as shown in FIG. 5, an example is taken where the collimating holes of each collimating hole group are classified into a plurality of rows in the longitudinal direction and the radioactive sources in the same row have the same longitude, and the collimating holes of each collimating hole group are classified into a plurality of rows in the latitudinal direction and the radioactive sources in the same row have the same latitude. Further, to implement non-coplanar radiation and to better protect normal tissues, in the source body provided by the present disclosure, the collimating holes have different positions in the latitudinal direction. That is, each collimating hole has different latitudes.

The collimator is provided with a plurality of collimating hole groups. For example, the collimator may be provided with two or more collimating hole groups. In FIG. 5, an exemplary description is made by taking an example where the collimator is provided with four collimating hole groups. Each collimating hole group includes a plurality of collimating holes, and the number of the plurality of collimating holes corresponding to the radioactive sources may range from 20 to 180, for example, 30 or 180. The present disclosure does not limit the number or arrangement of the collimating holes, and the example as shown in FIG. 5 merely serve as an exemplary description. The radiotherapy device also includes a plurality of radioactive sources, and the collimating holes on the collimator correspond to the radioactive sources in number and arrangement, such that the beams emitted from the radioactive sources intersect at a common focus after passing through the collimating holes.

For example, as shown in FIG. 6, the collimator also may be tube-shaped as shown in FIG. 6, and the longitudinal direction of the collimator is the direction as shown by Arrow j2 in FIG. 6. Two ends of a round table in FIG. 6 have equal size, or of course, may have different sizes (as shown in FIG. 10). The present disclosure does not limit the specific shape of the source body, and the shapes as shown in FIG. 5 and FIG. 6 serve as examples. An exemplary description is made in FIG. 6 by taking an example where the collimator is provided with two collimating hole groups and each collimating hole group includes 20 collimating holes.

The collimator 212 provided by the present disclosure also includes a shield region 2121 configured to shield the beams emitted from the plurality of radioactive sources. That is, the radioactive sources may be turned off by shielding rays from the radioactive sources with the collimator. The present disclosure does not limit the specific position of the shield region in the shield body. In FIG. 5, an exemplary description is made by taking an example where the shield region is spatially opposite to each collimating hole group.

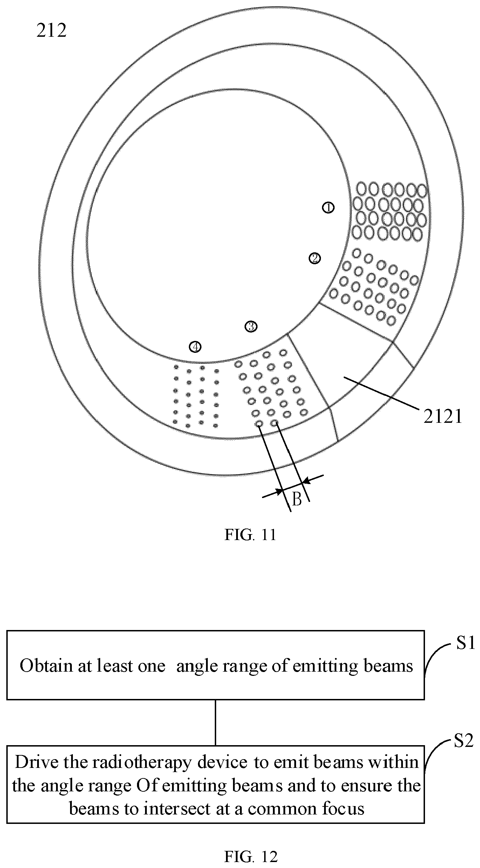

For example, in the collimator provided by the present disclosure, the shield region is located between any two adjacent collimating hole groups among the plurality of collimating hole groups. For example, as shown in FIG. 11, an exemplary description is made by taking an example where the shield region is located between the No. 2 collimating hole group and the No. 3collimating hole group.

In FIG. 11, an example is made where only one shield region is included. The present disclosure provides a collimator, which includes a plurality of shield regions. For example, the shield region may also be arranged between the No. 1collimating hole group and the No. 2collimating hole group. Alternatively, the shield region may also be arranged between the No. 3collimating hole group and the No. 4collimating hole group. Alternatively, the shield region may also be arranged between any two adjacent collimating hole groups. The present disclosure does not limit the number and distribution of the plurality of shield regions, and merely uses the above example to make an exemplary description.

The collimator provided by the present disclosure includes an internal collimator and an external collimator which are fixedly connected to one another, and a collimating hole on the internal collimator and a collimating hole on the external collimator are correspondingly arranged. That is, the collimator may include a double layer, and the internal collimator and the external collimator may be fixed through screw fastening.

The collimator provided by the present disclosure includes an internal collimator and an external collimator, which can rotate with respect to each other. For example, during the treatment, if an accident occurs, the radioactive source can be turned off quickly through the internal collimator, and then the external collimator is rotated to align the shield with the radioactive source to shield the radioactive source. Furthermore, the shield region of the internal collimator is aligned with the radioactive source, such that the radioactive source can be turned off completely.

For the collimator provided by the present disclosure, the collimating hole on the internal collimator is a taper hole, and/or the collimating hole on the external collimator is a straight hole. For example, the internal collimator and the external collimator each may be provided with a straight hole; or the internal collimator is provided with a taper hole, whereas the external collimator is provided with a straight hole; or the internal collimator and the external collimator each may be provided with a taper hole.

For the collimator provided by the present disclosure, the shield region is provided with a shield body, and a material density of the shield body is greater than that of the collimator. For example, the shield body is fixedly connected to the collimator, and the shield body may be formed by a tungsten block or a lead block or an alloy thereof. The collimating may be formed by cast iron. Thus, the shield body can better shield the radioactive source.

An embodiment not covered by the claimed invention provides a control driving method for a radiotherapy device. As shown in FIG. 12, the control driving method includes:
Step S1: obtaining at least one angle range of emitting beams; and
Step S2: driving the radiotherapy device to emit beams within the at least one angle range of emitting beams and to ensure the beams to intersect at a common focus.

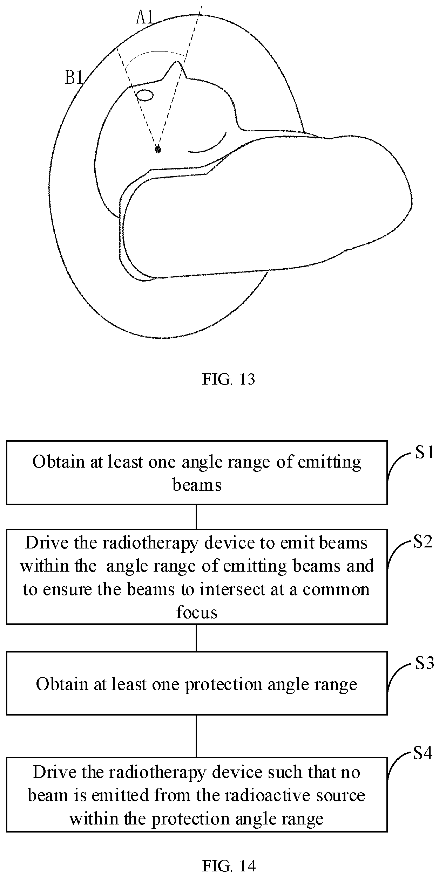

It is to be noted that a preset zero position is generally set for the driving apparatus in the radiotherapy device, and the zero position serves as a reference during the radiation treatment to confirm to drive within a drive angle range. In the present disclosure, the angle range of emitting beams may be an angle range required for the radiotherapy device to emit beams for radiation treatment, wherein the angle range is included in a corresponding treatment plan worked out by a physician according to a tumor image of a patient, and the angle range is an angle range for the driving apparatus to drive. For example, as shown in FIG. 13, in the corresponding treatment plan worked out by the physician according to the tumor image of the patient, the radiotherapy device performs a radiation treatment in a B1 interval, but does not perform the radiation treatment in an A1 interval (an A1 region includes radiation regions of two eyes to prevent rays from causing damage to optic nerves). The angle range of emitting beams is a drive angle range where the driving apparatus drives the radioactive source to radiate in the B1 interval, and a protection angle range is a drive angle range where the driving apparatus drives the radioactive source not to radiate in the A1 interval. During the radiation treatment, it is only necessary to rotate to irradiate within the drive angle range of the irradiation in the B1 interval, such that sensitive tissues may be prevented from damaging due to irradiating the eyes. For example, the drive angle range is a rotation angle of the motor. In the present disclosure, if the radiotherapy device rotates more than 360°, the drive angle range also exceeds 360°. Alternatively, if the radiotherapy device rotates more than 360°, the number of rotations and the drive angle range corresponding to different numbers of rotations are demarcated.

Of course, during the radiation treatment, rotatory irradiation may also be performed on the A1 region and the B1 region. In this case, the angle range of emitting beams is the drive angle range for irradiation in the A1 interval and the B1 region. For example, the angle range of emitting beams may be 360°. At this moment, irradiation dosage of sensitive tissues such as the optic nerves may be reduced by reducing irradiation time to protect the sensitive tissues and organs.

According to a control driving method provided by an embodiment not covered by the claimed invention, a radiotherapy device includes a plurality of radioactive sources, and source points of the plurality of radioactive sources are within a preset angle range in a longitudinal direction. The control driving method includes: obtaining at least one angle range of emitting beams; and driving the radiotherapy device to emit beams within the angle range of emitting beams and to ensure the beams to intersect at a common focus, such that sensitive tissues and organs such as eyes can be protected from extra damage during the treatment of head tumors.

As shown in FIG. 14, the control driving method provided by the embodiment not covered by the claimed invention further includes:
Step S3: obtaining at least one protection angle range. The at least one protection angle range is less than 360°.

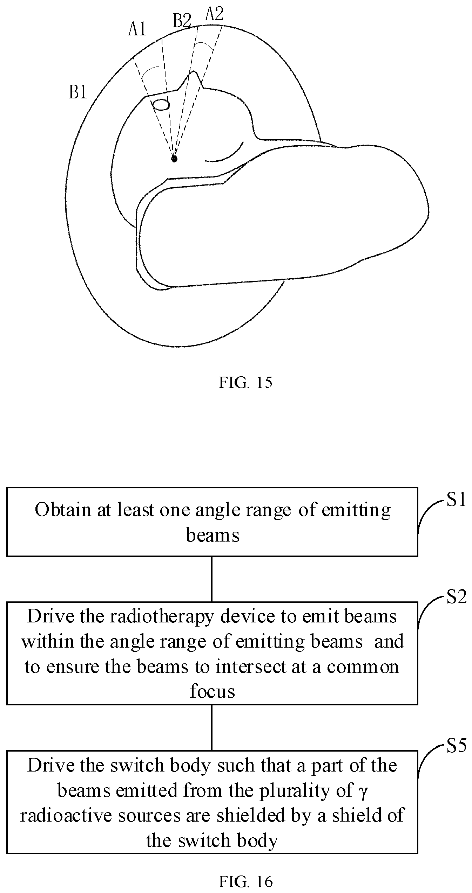

As shown in FIG. 15, the radiotherapy device performs a radiation treatment in a B1 interval and a B2 interval, but does not perform the radiation treatment in an A1 interval and an A2 interval (the A1 interval and the A2 interval correspond to eye regions to prevent rays from causing damage to optic nerves). The angle range of emitting beams is a drive angle range where the driving apparatus drives the radioactive source to radiate in the B1 interval and the B2 interval, and the protection angle range is a drive angle range where the driving apparatus drives the radioactive source not to radiate in the A1 interval or the A2 interval.

Step S4: driving the radiotherapy device such that no beam is emitted from the radioactive source within the protection angle range.

According to a control driving method provided by the embodiment not covered by the claimed invention, a radiotherapy device includes a plurality of radioactive sources, and source points of the plurality of radioactive sources are within a preset angle range in a longitudinal direction. The control driving method includes: obtaining at least one angle range of emitting beams and at least one protection angle range; and driving the radiotherapy device to emit beams within the angle range of emitting beams and to ensure the beams to intersect at a common focus, such that no beam is emitted from the radioactive sources within the protection angle range. In this way, during the treatment of head tumors, the sensitive tissues and organs such as eyes can be protected from extra damage.

For example, at least one of the angle range of emitting beams is adjacent to at least one of the protection angle range. As shown in FIG. 15, a radiation treatment is performed in the B1 interval and the B2 interval, but the radiation treatment is neither performed in the A1 interval nor performed in the A2 interval. Because the B1 interval is adjacent to the A1 interval, the angle range of emitting beams corresponding to the B1 interval is adjacent to the protection angle range corresponding to the A1 interval.

According to the control driving method provided by the embodiment not covered by the claimed invention, a plurality of angle range of emitting beams are obtained, and the radiotherapy device operates at different speeds within the at least two angle range of emitting beams. For example, referring to FIG. 15, when the radiation treatment is performed in the B1 interval and the B2 interval, the angle range of emitting beams corresponding to the B1 interval and the angle range of emitting beams corresponding to the B2 interval are obtained. The speed of the radiotherapy device within the angle range of emitting beams corresponding to the B1 interval is denoted as V1, and the speed of the radiotherapy device within the angle range of emitting beams corresponding to the B2 interval is denoted as V2, V1≠V2. Radiation time at different positions may be adjusted by adjusting the speed, such that dosage of the focus is adjusted.

For example, as shown in FIG. 13, when a rotatory radiation is performed in the A1 region and the B1 region during the radiation treatment, the angle range of emitting beams is the drive angle range for radiation performed in the A1 region and the B1 region. The speed of the radiotherapy device within the angle range of emitting beams corresponding to the B1 interval is denoted as V1, and the speed of the radiotherapy device within the angle range of emitting beams corresponding to the A1 interval is denoted as V2, V1 <V2. That is, the speed in the A1 interval is greater than the speed in the B1 interval, such that the dosage received by sensitive tissues in the A1 interval is reduced to protect the sensitive tissues and organs.

It is to be noted that the drive angle range in the present disclosure is the rotation angle of the motor, and the drive angle range also exceeds 360°. For example, if the rotation angle of the motor exceeds 360°, the number of rotations and the drive angle range corresponding to different numbers of rotations are demarcated. The radiotherapy device operates at different speeds within at least two angle range of emitting beams, and has different drive speeds in the same radiation interval corresponding to different numbers of rotations. For example, if planned treatment time for a radiation treatment is 2min, and 1min is required for per rotation of the motor, as shown in FIG. 15, the drive speed for radiation in the B1 region within the angle range of emitting beams of the first rotation is denoted as V1, and the drive speed for radiation in the B1 region within the angle range of emitting beams of the second rotation is denoted as V2, V1≠V2.

According to the control driving method provided by an embodiment not covered by the claimed invention, for example, as shown in above figures, two angle range of emitting beams having different speeds are adjacent to each other.

According to the control driving method provided by an embodiment not covered by the claimed invention, the radiotherapy device is driven to reciprocate within the angle range of emitting beams. For example, if only one angle range of emitting beams is obtained, the radiotherapy device may reciprocate within the angle range of emitting beams to increase the dosage received by a tumor. Of course, if a plurality of angle ranges of emitting beams are obtained, the radiotherapy device may also reciprocate within the angle ranges of emitting beams to increase the dosage received by the tumor.

According to the control driving method provided by an embodiment not covered by the claimed invention, the shield body is provided with a shield region, Step S4 in FIG. 14 specifically includes: driving the radiotherapy device such that the beams emitted from the plurality of radioactive sources are shielded by the collimator. For example, the source body may be driven to misalign the collimator, such that the radioactive source on the source body is shielded by a pitch between the collimating holes on the collimator. A smaller angle of misaligned rotation is needed such that the source body misaligns the collimator, and thus the radioactive source can be quickly turned on or off. Alternatively, the source body may be driven to misalign the collimator, such that the radioactive source on the source body is shielded by the shield region on the collimator.

According to the control driving method provided by an embodiment not covered by the claimed invention, when the radiotherapy device further includes a switch body, taking steps in FIG. 14 as examples, as shown in FIG. 16, the control driving method further includes:

Step S5: driving the switch body such that a part of the beams emitted from the plurality of γ radioactive sources are shielded by a shield region of the switch body.

The switch body is as shown in FIG. 11. The switch body is driven such that a part of the beams emitted from the plurality of γ radioactive sources are shielded by the shield region of the switch body. In this way, a part of the beams emitted from the radioactive sources may be shielded by the shield region of the switch body, thereby implementing the objective of regulating radiation dose.

According to the control driving method provided by an embodiment not covered by the claimed invention, in the longitudinal direction, a pitch between two adjacent collimating holes in the same collimating hole group is larger than a size of the radioactive source. Step S4 in FIG. 14 specifically includes: driving the radiotherapy device such that the plurality of radioactive sources misalign with the collimating hole, wherein the beams emitted from a part of the radioactive sources are shielded by an edge region of the collimating hole group, and the beams emitted from the remaining radioactive sources are shielded by a pitch region between the collimating holes. For example, the source body may be driven to misalign the collimator, such that the radioactive sources on the source body are shielded by a pitch between the collimating holes on the collimator. A smaller angle of misaligned rotation is needed such that the source body misaligns the collimator, and thus radioactive sources can be quickly turned on or off.

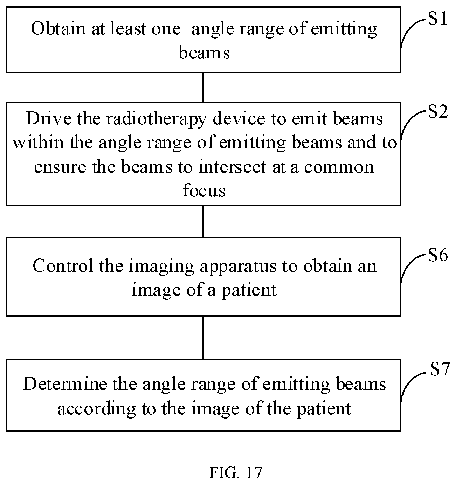

For example, in the radiotherapy device as shown in FIG. 8-FIG. 10, the common focus is located outside an end surface of the radioactive source apparatus. The radiotherapy device further includes an imaging apparatus, and the common focus is located within an imaging region of the imaging apparatus. As shown in FIG. 17, the control driving method also includes:
Step S6: controlling the imaging apparatus to obtain an image of a patient.
Step S7: determining the angle range of emitting beams according to the image of the patient.

It is to be noted that the angle range of emitting beams in Step S1 may be a angle range of emitting beams determined by a physician based on the image of the patient before the radiation treatment, and during the treatment, the angle range of emitting beams may be determined or adjusted based on the obtained image.

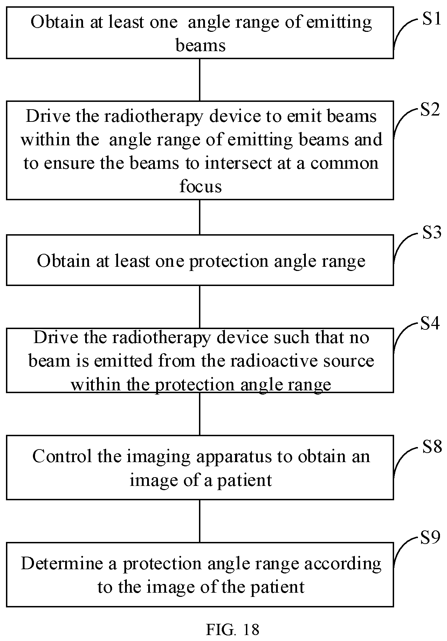

For example, the radiotherapy device as shown in FIG. 8-FIG. 10 further includes an imaging apparatus, and the common focus is located within an imaging region of the imaging apparatus. As shown in FIG. 18, the control driving method also includes:
Step S8: controlling the imaging apparatus to obtain an image of a patient; and
Step S9: determining a protection angle range according to the image of the patient.

Similarly, the protection angle range in Step S3 may be a protection angle range determined by the physician based on the image of the patient before the radiation treatment, and during the treatment, the protection angle range may be determined or adjusted based on the obtained image.

It is to be noted that in the control driving method provided by an embodiment not covered by the claimed invention, the present disclosure does not limit sequences of the above steps, and merely uses the above examples to make an exemplary description.

The embodiments set forth above are only illustrated as alternative embodiments of the present disclosure, and are not intended to limit the present disclosure.

## Claims

1. A radiotherapy device for treating head tumor, comprising a radioactive source apparatus (21), wherein the radioactive source apparatus comprises a source body (211), a shielding apparatus (24) and a collimator (212), and the source body (211) is provided with a plurality of radioactive sources (211a), wherein the collimator (212) is provided with a plurality of collimating hole groups, and an included angle (A) of each of the plurality of collimating hole groups in a longitudinal direction is within a preset included angle range; and each of the plurality of collimating hole groups comprises a plurality of collimating holes, and beams emitted from the plurality of radioactive sources intersect at a common focus (f) after passing through each collimating hole of the collimating hole group, **characterized in that** the common focus (f) is located outside an end surface of the radioactive source apparatus (21), the shielding apparatus (24) is located at a side of the radioactive source apparatus (21), and beams passing through the common focus (f) are shielded by the shielding apparatus (24) during treatment.

2. The radiotherapy device according to claim 1, wherein the preset included angle range is between 5° and 60°.

3. The radiotherapy device according to claim 1, wherein the included angle range of each of the collimating hole groups in a latitude direction is between 20° and 60°.

4. The radiotherapy device according to claim 1, wherein the collimator (212) further comprises a shield region (2121) configured to shield the beams emitted from the plurality of radioactive sources (211a).

5. The radiotherapy device according to claim 1, wherein the collimator (212) comprises an inner collimator and an outer collimator that are fixedly connected.

6. The radiotherapy device according to claim 5, wherein collimating holes on the inner collimator and collimating holes on the outer collimator are arranged correspondingly; wherein the collimating holes on the inner collimator are tapered holes, and/or the collimating holes on the outer collimator are straight holes.

7. The radiotherapy device according to claim 1, wherein the shielding apparatus (24) is an annular body.

8. The radiotherapy device according to claim 7, wherein the radiotherapy device further comprises an imaging apparatus (23), the imaging apparatus (23) is arranged at a side of the radioactive source apparatus (21), and the common focus (f) is located within an imaging region of the imaging apparatus (23).

9. The radiotherapy device according to claim 1, wherein the radiotherapy device further comprises a treatment couch (22), and the shielding apparatus (24) is provided with a channel for facilitating movement of the treatment couch (22).

10. The radiotherapy device according to claim 1, wherein the shielding apparatus (24) is a shielding block that can rotate along a central axis of the source body (211) to follow the rotation of a radioactive source (211a) provided in the radioactive source apparatus (21) so as to receive the beams having passed through the common focus (f).

## Patentansprüche

1. Strahlentherapiegerät zur Behandlung eines Kopftumors, umfassend eine radioaktive Quellenvorrichtung (21), wobei die radioaktive Quellenvorrichtung einen Quellenkörper (211), eine Abschirmvorrichtung (24) und einen Kollimator (212) umfasst und der Quellenkörper (211) mit mehreren radioaktiven Quellen (211a) versehen ist, wobei der Kollimator (212) mit mehreren Kollimationslochgruppen versehen ist und ein eingeschlossener Winkel (A) jeder der mehreren Kollimationslochgruppen in einer Längengradrichtung innerhalb eines voreingestellten eingeschlossenen Winkelbereichs liegt; und wobei jede der mehreren Kollimationslochgruppen mehrere Kollimationslöcher umfasst und sich Strahlen, die von den mehreren radioaktiven Quellen emittiert werden, nach ihrem Durchgang durch jedes Kollimationsloch der Kollimationslochgruppe in einem gemeinsamen Brennpunkt (f) schneiden, **dadurch gekennzeichnet, dass** sich der gemeinsame Brennpunkt (f) außerhalb einer Endfläche der radioaktiven Quellenvorrichtung (21) befindet, die Abschirmvorrichtung (24) auf einer Seite der radioaktiven Quellenvorrichtung (21) angeordnet ist und Strahlen, die durch den gemeinsamen Brennpunkt (f) durchgehen, während der Behandlung durch die Abschirmvorrichtung (24) abgeschirmt werden.

2. Strahlentherapiegerät nach Anspruch 1, wobei der voreingestellte eingeschlossene Winkelbereich zwischen 5° und 60° liegt.

3. Strahlentherapiegerät nach Anspruch 1, wobei der Bereich des eingeschlossenen Winkels jeder der Kollimationslochgruppen in einer Breitengradrichtung zwischen 20° und 60° liegt.

4. Strahlentherapiegerät nach Anspruch 1, wobei der Kollimator (212) ferner einen Abschirmbereich (2121) umfasst, der dazu konfiguriert ist, die von den mehreren radioaktiven Quellen (211a) emittierten Strahlen abzuschirmen.

5. Strahlentherapiegerät nach Anspruch 1, wobei der Kollimator (212) einen inneren Kollimator und einen äußeren Kollimator umfasst, die fest miteinander verbunden sind.

6. Strahlentherapiegerät nach Anspruch 5, wobei Kollimationslöcher am inneren Kollimator und Kollimationslöcher am äußeren Kollimator korrespondierend zueinander angeordnet sind; wobei die Kollimationslöcher am inneren Kollimator konische Löcher sind und/oder die Kollimationslöcher am äußeren Kollimator gerade Löcher sind.

7. Strahlentherapiegerät nach Anspruch 1, wobei die Abschirmvorrichtung (24) ein ringförmiger Körper ist.

8. Strahlentherapiegerät nach Anspruch 7, wobei das Strahlentherapiegerät ferner eine Bildgebungsvorrichtung (23) umfasst, die Bildgebungsvorrichtung (23) auf einer Seite der radioaktiven Quellenvorrichtung (21) angeordnet ist und sich der gemeinsame Brennpunkt (f) innerhalb eines Bildgebungsbereichs der Bildgebungsvorrichtung (23) befindet.

9. Strahlentherapiegerät nach Anspruch 1, wobei das Strahlentherapiegerät ferner eine Behandlungsliege (22) umfasst, und die Abschirmvorrichtung (24) mit einem Kanal zur Erleichterung der Bewegung der Behandlungsliege (22) versehen ist.

10. Strahlentherapiegerät nach Anspruch 1, wobei die Abschirmvorrichtung (24) ein Abschirmblock ist, der sich entlang einer zentralen Achse des Quellenkörpers (211) drehen kann, um der Drehung einer radioaktiven Quelle (211a), die in der radioaktiven Quellenvorrichtung (21) vorgesehen ist, zu folgen und so die Strahlen, die durch den gemeinsamen Brennpunkt (f) hindurchgetreten sind, aufzunehmen.

## Revendications

1. Dispositif de radiothérapie pour le traitement de la tumeur au cerveau, comprenant un appareil à source radioactive (21), dans lequel l'appareil à source radioactive comprend un corps de source (211), un appareil de blindage (24) et un collimateur (212), et le corps de source (211) est pourvu d'une pluralité de sources radioactives (211a), dans lequel le collimateur (212) est pourvu d'une pluralité de groupes de trous de collimation, et un angle inclus (A) de chacun de la pluralité de groupes de trous de collimation dans une direction longitudinale se trouve dans une plage prédéfinie d'angle inclus ; et chacun de la pluralité de groupes de trous de collimation comprend une pluralité de trous de collimation, et des faisceaux émis par la pluralité de sources radioactives se croisent à un foyer commun (f) après avoir passé par chaque trou de collimation du groupe de trous de collimation, **caractérisé en ce que** le foyer commun (f) est situé à l'extérieur d'une surface d'extrémité de l'appareil à source radioactive (21), l'appareil de blindage (24) est situé sur un côté de l'appareil à source radioactive (21), et des faisceaux passant par le foyer commun (f) sont blindés par l'appareil de blindage (24) pendant le traitement.

2. Dispositif de radiothérapie selon la revendication 1, dans lequel la plage prédéfinie d'angle inclus est de 5° à 60°.

3. Dispositif de radiothérapie selon la revendication 1, dans lequel la plage d'angle inclus des groupes de trous de collimation dans une direction de latitude est comprise entre 20° et 60°.

4. Dispositif de radiothérapie selon la revendication 1, dans lequel le collimateur (212) comprend en outre une région de blindage (2121) configurée pour blinder les faisceaux émis par la pluralité de sources radioactives (211a).

5. Dispositif de radiothérapie selon la revendication 1, dans lequel le collimateur (212) comprend un collimateur interne et un collimateur externe qui sont reliés de manière fixe.

6. Dispositif de radiothérapie selon la revendication 5, dans lequel les trous de collimation sur le collimateur interne et les trous de collimation sur le collimateur externe sont agencés en correspondance ; dans lequel les trous de collimation sur le collimateur interne sont des trous coniques, et/ou les trous de collimation sur le collimateur externe sont des trous droits.

7. Dispositif de radiothérapie selon la revendication 1, dans lequel'appareil de blindage (24) est un corps annulaire.

8. Dispositif de radiothérapie selon la revendication 7, dans lequel le dispositif de radiothérapie comprend en outre un appareil d'imagerie (23), l'appareil d'imagerie (23) étant agencé sur un côté de l'appareil à source radioactive (21), et le foyer commun (f) étant situé dans une région d'imagerie de l'appareil d'imagerie (23).

9. Dispositif de radiothérapie selon la revendication 1, dans lequel le dispositif de radiothérapie comprend en outre une table de traitement (22), et l'appareil de blindage (24) est pourvu d'un canal pour faciliter le déplacement de la table de traitement (22).

10. Dispositif de radiothérapie selon la revendication 1, dans lequel l'appareil de blindage (24) est un bloc de blindage configuré pour tourner le long d'un axe central du corps de source (211) pour suivre la rotation d'une source radioactive (211a) prévue dans l'appareil à source radioactive (21) afin de recevoir les faisceaux ayant passé par le foyer commun (f).
